(19) Europäisches Patentamt / European Patent Office / Office européen des brevets

(11) **EP 2 297 184 B1**

(12) **EUROPEAN PATENT SPECIFICATION**

(45) Date of publication and mention
of the grant of the patent:
**04.09.2013 Bulletin 2013/36**

(51) Int Cl.:
*C07K 14/00* (2006.01)   *C07K 17/04* (2006.01)
*C07K 1/14* (2006.01)   *C07K 1/18* (2006.01)

(21) Application number: **09751237.0**

(22) Date of filing: **15.05.2009**

(86) International application number:
**PCT/US2009/044113**

(87) International publication number:
**WO 2009/143008 (26.11.2009 Gazette 2009/48)**

(54) **SOLUBILIZATION OF PROTEINS IN REVERSE MICELLES BY EXTRACTION FROM A SOLID SUPPORT**

SOLUBILISIERUNG VON PROTEINEN IN REVERSEN MIZELLEN DURCH EXTRAKTION AUS EINEM FESTEN TRÄGER

SOLUBILISATION DE PROTÉINES EN MICELLES INVERSES PAR EXTRACTION À PARTIR D'UN SUPPORT SOLIDE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **21.05.2008 US 54941**

(43) Date of publication of application:
**23.03.2011 Bulletin 2011/12**

(73) Proprietor: **Daedalus Innovations Llc**
**Philadelphia, PA 19104 (US)**

(72) Inventor: **PETERSON, Ronald, W.**
**Media**
**PA 19063 (US)**

(74) Representative: **Leissler-Gerstl, Gabriele**
**Hoefer & Partner**
**Patentanwälte**
**Pilgersheimer Strasse 20**
**81543 München (DE)**

(56) References cited:
WO-A1-94/09365    US-A- 4 839 419
US-B1- 6 486 672

- **MATZKE S F ET AL: "MECHANISMS OF PROTEIN SOLUBILIZATION IN REVERSE MICELLES", BIOTECHNOLOGY AND BIOENGINEERING, WILEY & SONS, HOBOKEN, NJ, US, vol. 40, no. 1, 1 January 1992 (1992-01-01), pages 91-102, XP000651477, ISSN: 0006-3592, DOI: 10.1002/BIT. 260400114**
- **EDUARDO P. MELO ET AL: "Cutinase-AOT interactions in reverse micelles: the effect of 1-hexanol", CHEMISTRY AND PHYSICS OF LIPIDS, vol. 124, no. 1, 1 June 2003 (2003-06-01), pages 37-47, XP55017798, ISSN: 0009-3084, DOI: 10.1016/S0009-3084(03)00031-8**
- **JOSHUA WAND A ET AL: "Chapter 6.4: Reverse micelle technology", PROTEIN NMR FOR THE MILLENIUM,, 1 January 2003 (2003-01-01), pages 130-137, XP008147899, ISBN: 978-0-306-47448-4 [retrieved on 2006-04-11]**
- **J B CHAUDHURI ET AL: "Recovery of proteins from reversed micelles using a novel ion-exchange material", BIOTECHNOLOGY TECHNIQUES, vol. 8, no. 12, 1 December 1994 (1994-12-01), pages 909-914, XP55017738,**
- **J. H. CHILL: "NMR study of the tetrameric KcsA potassium channel in detergent micelles", PROTEIN SCIENCE, vol. 15, no. 4, 1 January 2006 (2006-01-01), pages 684-698, XP55017790, ISSN: 0961-8368, DOI: 10.1110/ps.051954706**

## Description

## Field of the Invention

The present invention relates to methods of using reverse micelles for solubilizing proteins.

## Background of the Invention

Biomedical research is a large component of the global economy. The emphasis on molecular intervention in disease using pharmaceutical agents is a cornerstone of modern medicine. Surprisingly, the drug discovery strategy remains much the way it has been for decades, though the speed with which new compounds are examined has dramatically accelerated. The impact of "rational" drug design is only now just emerging. Knowledge of the molecular structure of potential therapeutic targets - most often proteins - is critical to the emerging rational drug design strategy and is a central component of leading-edge pharmaceutical research.

New drugs typically are developed by starting with a large pool of compounds and performing random screens against a potential target. From a pool of 10,000 compounds only about five will make it to clinical trials. This process is inefficient and costly; usually about one third of the total development cost is spent here. It has been known that structural information about the targets of these drugs can greatly enhance the discovery and development process, thus lowering the costs and development time for new medicines. Hence there is justification for funding national programs such as the Protein Structure Initiative to get this information into the public domain and make it available to all researchers.

The determination of protein structures is a vital component of our understanding of nature. In the area of medicine and drug design the structure of a protein is necessary for developing effective pharmaceuticals. There are two techniques that dominate the structure determination field. One is x-ray crystallography and the other is nuclear magnetic resonance (NMR) spectroscopy. There are of course advantages and disadvantages to both. X-ray crystallography is the oldest of the techniques and therefore is the best established in terms of methods for collecting and analyzing data. A structure determined by x-ray crystallography is a largely static picture of the protein molecule. This method has few limitations on the protein provided it can be crystallized. Therein lies one major disadvantage of this technique. It turns out that the biggest bottleneck in this method is the generation of crystals suitable for analysis. The vast majority of the potential proteins targeted for drug development are not currently amenable to this technique due to the present lack of a method for making the crystal sample.

An alternate approach to obtaining high resolution structures of proteins is nuclear magnetic resonance (NMR). NMR employs the same basic physical principles that underlies the commonly recognized medical imaging technique known as Magnetic Resonance Imaging or MRI. NMR is a powerful technique that can provide the structure of a protein in an aqueous, thus more natural, environment as opposed to a crystal. Proteins are not static entities; rather they are dynamic. It is becoming increasingly clear that the dynamics of the protein will provide important information for refining drug design and our understanding of proteins in general. Protein sample preparation is typically less of a problem for NMR, although special reagents are necessary which add to the cost of the technique. One major disadvantage of NMR with respect to x-ray crystallography is the cost of the instrumentation and the requirement for highly skilled labor to operate the NMR spectrometer and analyze the results. Another disadvantage is that current NMR techniques can only be easily applied to proteins of limited size and many proteins of interest are outside of this range. However, new tools are reducing this limitation and NMR is experiencing a resurgence as a structure determination tool.

Solution NMR spectroscopy continues to play a central role in the characterization of the structure and dynamics of proteins, nucleic acids and their complexes. Over the past fifteen years there have been remarkable developments in NMR techniques and supporting technologies such that the comprehensive structural characterization of proteins of moderate size (about 30 kDa) has become routine (for concise reviews, see Clore, G.M., and Gronenborn, A.M. 1997. Nat. Struct. Biol. 4 Suppl: 849-853; Wagner, G., 1997, Nat. Struct. Biol. 4 Suppl: 841-844). The size of protein structures that can be solved by modern NMR techniques has dramatically increased over the past decade. Coupled with the introduction of heteronuclear (Sorensen, O.W., Eich, G.W., Levitt, M.H., Bodenhausen, G., and Ernst, R.R. 1987. Prog. NMR Spectr. 16: 163-192) and ultimately triple resonance spectroscopy (Kay, L.E., Clore, G.M., Bax, A., and Gronenbom, A.M. 1990. Science 249: 411-414), was the widespread use of recombinant technologies in order to introduce NMR-active isotopes into proteins and nucleic acids (McIntosh, L.P., and Dahlquist, F.W. 1990. O. Rev. Biophys. 23: 1-38; LeMaster, D.M. 1994, Prog. NMR Spectr. 26: 371-419). With the development of multinuclear and multidimensional capabilities, NMR is now able to efficiently and comprehensively deal with proteins of significant size and spectral complexity and has, with the advent of Transverse Relaxation Optimized Spectroscopy, or TROSY (Pervushin, K., Riek, R., Wider, G., and Wuthrich, K. 1997. Proc. Nat. Acad. Sci. USA 94: 12366-12371), reached significant heights (Riek, R., Pervushin, K., and Wuthrich, K. 2000. Trends Biochem. Sci. 25: 462-468; Tugarinov, V., Muhandiram, R., Ayed, A., and Kay, L.E. 2002. J. Am. Chem. Soc. 124: 10025-10035).

However, increasing protein size brings with it several important limitations that unfortunately compound each other to often restrict the size of a protein that can be efficiently approached by modern NMR techniques. First, increasing size leads to slower tumbling and correspondingly shorter spin-spin relaxation times. Thereby the fundamental techniques of protein NMR spectroscopy, triple resonance and total correlation technologies, begin to fail. As lines broaden, basic sensitivity also begins to become a limiting issue. Second, increasing size leads to increasingly complex spectra. Spectral degeneracy complicates the assignment process and renders assignment of Nuclear Overhauser Effects (NOEs) and other structural restraints to parent nuclei problematic.

Briefly stated, increasing size leads to slower tumbling of the macromolecule which in turn results in more efficient dipolar relaxation processes and shorter spin-spin relaxation times. The coherence transfer processes underlying current triple resonance-based assignment strategies are time-dependent and begin to fail with proteins that are about 30 kiloDaltons (kD) and larger. Chemical approaches such as random partial- or perdeuteration have been used successfully to reduce the dipolar field so that high resolution $^{15}$N Heteronuclear Single Quantum Coherence, or $^{15}$N-HSQC, spectra can be obtained (LeMaster, D.M. 1994. Prog. NMR Spectr. 26: 371-419.). Unfortunately, perdeuteration also drastically limits the structural information available from the NOE. Fractional deuteration has limited sensitivity, and its applicability as a general solution to the dipolar broadening displayed by proteins above 35 kDa is uncertain. Spectroscopic solutions to these problems are also appearing. Some find their roots in the steady improvement in the use of the rotating frame to provide for more efficient isotropic mixing for coherence transfer. These and other recent advances such as TROSY are extremely powerful, but they do not solve all of the issues facing the solution NMR spectroscopist. The difficulty of dealing comprehensively with large proteins in a general manner remains as a significant limitation to applying solution NMR methods to the rapidly growing list of proteins being discovered by the molecular biology community.

NMR Spectroscopy of Encapsulated Proteins Dissolved in Low Viscosity Fluids.

In order to improve tumbling and spin-spin relaxation times, a solvent having a lower viscosity than that of water may be used. The basic idea is to take advantage of the linear dependence for molecular reorientation on the bulk solvent viscosity as related by the classic Stokes-Einstein relationship for a sphere:

$$\tau_m = \frac{\eta V}{kT}$$

where $\tau_m$ is the molecular reorientation corre-

lation time, $\eta$ is the bulk solvent viscosity, $V$ is the volume of the sphere, $k$ is the Boltzmann constant and $T$ is the absolute temperature. Though more complex treatments are available, the Stokes-Einstein relationship serves to illustrate the approach. By encapsulating a protein within the protective shell of a reverse micelle one can solubilize the resulting particle in a very low viscosity solvent, for example, liquid pentane, butane, propane or ethane.

Such solvents are incompatible with proteins. However, by encapsulating a protein in reverse micelles, it can be safely dissolved in such solvents without denaturation. Wand and coworkers have developed and demonstrated the basic approach using the small, stable, water-soluble protein ubiquitin (Wand, A.J., Ehrhardt, M.R., and Flynn, P.F. 1998. Proc. Nat. Acad. Sci. USA 95: 15299-15302), the structure of which was determined to very high resolution inside the reverse micelle, and was shown to match that found in aqueous solution (Babu, C.R., Flynn, P.F., and Wand, A.J. 2001. J. Am. Chem. Soc. 123: 2691-2692). This technique has since been expanded to include a variety of other soluble proteins (Lefebvre, B.G., Liu, W., Peterson, R.W., Lefebvre, B.G., and Wand, A.J. 2005, J. Magn. Reson., 175: 158-162; Peterson, R.W., Lefebvre, B.G., and Wand, A.J. 2005, J. Am. Chem. Soc. 127: 10176-10177), using surfactants other than AOT (Lefebvre, B.G., Liu, W., Peterson, R.W., Valentine, K.G., and Wand, A.J. 2005. J. Magn. Reson. 175: 158-162; Peterson, R.W., Pometun, M.S., Shi, Z., and Wand, A.J. 2005. Protein Sci. 14: 2919-2921; Shi, Z., Peterson, R.W., and Wand, A.J. 2005. Langmuir 21: 10632-10637). In addition it has been shown that proteins inside a reverse micelle in liquid ethane do in fact tumble faster than they do in water, even for small proteins such as ubiquitin (Peterson, R.W., Lefebvre, B.G., and Wand, A.J. 2005, J. Am. Chem. Soc. 127: 10176-10177).

In addition to structure determination it has become apparent that a persistent problem in drug discovery is just trying to maintain stable constructs of proteins for biochemical assays. Certain proteins such as membrane proteins, that is, proteins that associate with cell membranes either by embedding in or anchoring to the membrane, and also metastable proteins are exceptionally difficult to handle. These proteins show a distinct tendency to aggregate in solution either as free protein or as a result of ligand binding before studies can be performed. This is a major problem in structure determination. Integral membrane proteins such as G-protein coupled receptors (GPCR) are a major target of current drug therapies, accounting for approximately 50% of the current total pharmaceutical market. The structure of the first therapeutic GPCR, the β2-adrenergic GPCR, was solved only in November of 2007 (Cherezov, V., Rosenbaum Daniel, M., Hanson Michael, A., Rasmussen Soren, G.F., Thian Foon, S., Kobilka Tong, S., Choi, H.-J., Kuhn, P., Weis William, I., Kobilka Brian, K., et al. 2007. Science (New York, N.Y.) 318: 1258-1265). Obviously new methods for handling such proteins are required.

Reverse micelles have a growing track record of being uniquely suited for solubilizing proteins of all classes, and provide a robust construct by which such proteins can be studied using a variety of spectroscopic techniques. However, one of the more pressing technical challenges is being able to achieve sufficient concentrations of the protein in the reverse micelle sample to do meaningful spectroscopy.

Methods for preparing reverse micelles

Reverse micelles will spontaneously form as transparent solutions in a low polarity liquid, and are thermodynamically stable assemblies of surfactant molecules organized around a water core. Reverse micelles were the subject of extensive attention in the 1980s as potential devices for a range of applications including separations, chromatography and reaction processes (Goklen, K.E., and Hatton, T.A. 1985. Biotechnol Progr 1: 69-74; Goklen, K.E., and Hatton, T.A. 1985. Abstr Pap Am Chem S 190: 128-Mbd). More recently, they have become the focus of further attention in the context of hosting various chemical reactions in solvents with low environmental impact such as supercritical carbon dioxide (Johnston, K.P., Randolph, T., Bright, F., and Howdle, S. 1996. Science 272: 1726).

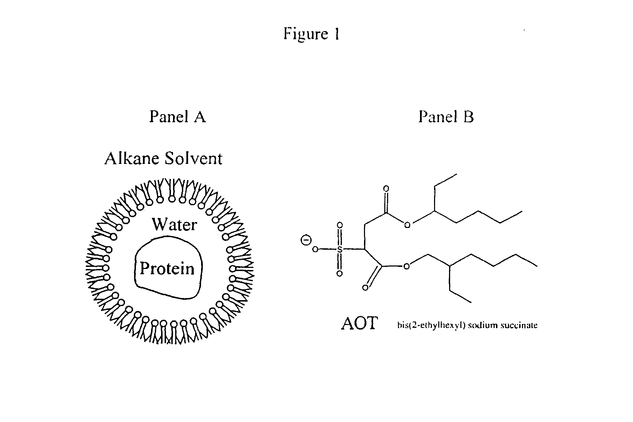

The size and stability of a reverse micelle is dependent upon the amount of water loading, the molar ratio of water to surfactant, $W_o$. The commonly used surfactant sodium bis(2-ethylhexyl)sulfosuccinate (AOT) has been extensively studied in this respect for a variety of organic solvents. The chemical structure of AOT is shown in Figure 1. Water loadings have been described that yield stable reverse micelles of AOT in a variety of long and short chain alkane solvents large enough to accommodate proteins (e.g., Frank, S.G., and Zografi, G. 1969. J. Coll. Interf. Sci. 29: 27-35; Gale, R.W., Fulton, J.L., and Smith, R.D. 1987. J. Am. Chem. Soc. 109: 920-921; Fulton, J.L., and Smith, R.D. 1988. Acs Sym Ser 373: 91-107; Fulton, J.L., Blitz, J.P., Tingey, J.M., and Smith, R.D. 1989. J Phys Chem-Us 93: 4198-4204). Indeed, there have been reports that all of the three shortest alkane solvents will support formation of a single phase containing reverse micelles. Ethane requires significantly elevated pressures (up to 650 bar, see Fulton, J.L., and Smith, R.D. 1988. Acs Sym Ser 373: 91-107; Fulton, J.L., Blitz, J.P., Tingey, J.M., and Smith, R.D. 1989. J Phys Chem-Us 93: 4198-4204; Smith, R.D., Fulton, J.L., Blitz, J.P., and Tingey, J.M. 1990. J Phys Chem-Us 94: 781-787) to obtain optimal behavior while butane requires the lowest pressure (a few bar).

As is known in the art, reverse micelles are typically prepared in one of two ways (Matzke, S.F. et al. 1992. Biotechnology and Bioengineering 40:91-102). Since water content is directly correlated to the size of the reverse micelle, and thus the performance in NMR spectroscopy experiments, the goal has been to minimize the amount of water present. To that end, the preferred method for preparing reverse micelles is to use a concentrated stock of the protein of interest, and inject that into a reverse micelle solution whereupon the protein is spontaneously encapsulated (e.g. Melo, E.P. et al., 2003 Chemistry and Physics of Lipids 124:37-47). For the purposes of sample preparation for NMR spectroscopy, this method requires the starting protein stock to be much greater than 1 mM in concentration. Concentration of the protein can be achieved by filtration methods in the aqueous phase or by other methods such as lyophilization and resuspension of the protein in a small aliquot of water or buffer. The ability to concentrate a protein in the aqueous phase to this degree is almost certainly not feasible for complex constructs such as membrane proteins, and in reality is more of an exception than the rule for most water soluble proteins as well. Lyophilizing integral membrane constructs has also been found to be disruptive to the construct in many cases. Thus it would appear that the applicability of either of the above concentration approaches is limited to special cases.

The alternative is to use a bulk transfer method whereby an aqueous phase containing the protein is brought into contact with an equal volume of organic phase containing empty reverse micelles. Upon mild agitation the protein spontaneously transfers into the organic phase in the form of encapsulated proteins; that is, reverse micelles. The water loading ($W_o$) tends to be much higher using this method; however, proper selection of buffer components makes the water content a manageable problem. Unfortunately, attempts to use this method with integral membrane proteins have proven difficult. The reason for this is not known, but it is thought that the driving force for encapsulation exclusively in the organic phase is overcome by the tendency for the protein to remain at the natural interface region between the aqueous and organic phases. Regardless of the reason, it has become apparent that this method for encapsulating proteins in reverse micelles generally fails for integral membrane proteins. However, the present invention has revealed that the solution to the problem of achieving higher protein concentrations in reverse micelle solutions for all classes of proteins is immobilization of the protein followed by extraction of the immobilized protein directly into a reverse micelle solution.

**Summary of the Invention**

The present invention relates to a general method for solubilizing proteins in a reverse micelle solution. The protein is first immobilized on a solid support, the hydration of the immobilized protein plus support is reduced, and then the protein is extracted directly into a reverse micelle organic phase. For more difficult proteins, for example integral membrane proteins, immobilization of the protein is facilitated using a carrier membrane detergent, which is subsequently exchanged for a surfactant capable of forming reverse micelles. Excess surfactant is eluted, the hydration of the immobilized protein is reduced and the protein is extracted directly into a reverse micelle organic phase, as above.

Proteins include, without limitation, soluble proteins, membrane-anchoring proteins, integral membrane proteins, and proteins that aggregate in solution.

The solid support may be any medium capable of binding and releasing the protein from the aqueous phase, including, without limitation, ion exchange resins and reversed phase media.

Surfactants capable of micelle formation include, without limitation, hexadecyl trimethylammonium bromide, dodecyl trimethylammonium bromide, sodium bis(2-ethylhexyl) sulfosuccinate, lauryldimethylamine oxide, and N,N-dimethyl-N,N-dihexadecyl ammonium bromide. A co-surfactant alcohol, for example hexanol, may also be added to obtain the reverse micelle organic solution.

The organic phase can be any solvent capable of supporting reverse micelles, for example and without limitation, decane, nonane, octane, iso-octane, heptane, hexane, pentane, butane, propane, ethane, or isomers or mixtures thereof.

Excess water may be removed from the immobilized protein by various methods, including and without limitation, draining the water from the solid support, evaporation at atmospheric or reduced pressure, and addition of an organic solvent to displace water.

The bulk solvent for carrying the surfactant used for the detergent exchange may be either water or an organic solvent.

The reverse micelle-solubilized protein may be subjected to spectroscopic analysis, for example and without limitation, NMR spectroscopy, fluorescence spectroscopy, UV-visible (UV-Vis) spectroscopy, and infrared (IR) spectroscopy. Small molecule and/or ligand binding, as well as protein-protein interactions may be studied using these techniques. For the binding and interaction studies, either the protein or the reverse micelle phase may contain the appropriate small molecules, natural or synthetic protein ligands, or other natural membrane components, for example cholesterol.

**Brief Description of the Drawings**

Figure 1, panel A depicts a schematic illustration of a reverse micelle containing a water soluble protein. Figure 1, panel B shows the chemical structure of AOT, one surfactant that is commonly used to construct reverse micelle systems having a significant internal aqueous core.

**Detailed Description of the Invention**

The present invention provides novel general methods for solubilization of proteins. The method includes the elution or extraction of the protein, which has been immobilized on a solid support, with a reverse micelle organic phase. The method provides protein reverse micelle solutions of sufficient purity and concentration to be useful for NMR and/or other spectroscopic analysis. A stepwise description and rationale of the method for preparing the protein reverse micelle solutions follows below.

The protein is immobilized on an ion-exchange chromatographic resin. This can be cationic, anionic, or any other binding resin used in protein purification. Typically the resin selected has an ion exchange group that matches the charge on the surfactant head-group. The binding conditions are selected so that the protein will remain bound during subsequent surfactant exchange and wash cycles.

If necessary, the surfactant is exchanged. For integral membrane proteins that have associated carrier membranes that keep the protein solubilized during the purification process, this step is often necessary. For other classes such as water soluble proteins, metastable proteins, and typical membrane-anchoring proteins, this step can be omitted. The membrane detergents used for purification are exchanged for surfactants suitable for forming reverse micelles. It does not require large quantities of the surfactant to competitively replace the bound detergents, as might be the case when using other techniques such as dialysis. Several other advantages such as reducing aggregation losses and overall faster surfactant exchange times, can be expected by making the exchange while the protein is immobilized.

The excess surfactants are washed away, and the hydration of the resin plus immobilized protein is reduced. The removal of excess water is necessary to control the final water content in the reverse micelle. Dehydration can be achieved by draining away excess water from the resin and placing the immobilized protein under vacuum, or excess water can be displaced using an organic solvent having low water solubility. The latter method of dehydration is achieved by washing the resin with an organic solvent. The selection of this solvent is critical; it should not be a solvent that can support reverse micelles by itself, nor one of sufficient hydrophobicity that it elutes the protein. Suitable solvents include, without limitation, diethyl ether, carbon disulfide, and methylene chloride. After washing with an organic solvent, the resin is dried to remove traces of the solvent.

The protein is extracted or eluted into the reverse micelle solution. The target buffer for the final reverse micelle sample is used to pre-form reverse micelles of the desired surfactant in the organic phase, and this solution is then brought into contact with the resin to initiate extraction and encapsulation of the membrane protein. The reverse micelle organic phase may be passed through a column of the resin containing the immobilized protein, in a manner commonly employed in protein purification or ion exchange chromatography. The collected organic phase which elutes from the column contains the protein in substantially pure form encapsulated in the reverse micelles. The term "substantially pure" signifies a protein purity of at least 98%. Alternatively the protein may be extracted from the resin by covering the resin with an aliquot of the reverse micelle organic phase and allowing it to stand at room temperature for a brief period of time, for example from about 5 to about 15 minutes. The resulting protein reverse micelle solution is either drawn off the resin, or centrifuged and collected. The extraction or elution process is essentially quantitative as demonstrated by UV-Vis spectroscopy.

Many proteins are amenable to the method of the present invention. These proteins include not only water-soluble proteins, but also the more intractable integral membrane proteins. Water-soluble proteins include almost all globular proteins, and many enzymes. Integral membrane proteins are embedded in the membrane of a cell or organelle. They require a detergent or bipolar solvent in order to be displaced, and are difficult to obtain in homogeneous form. Integral membrane proteins include transmembrane proteins and integral monotopic proteins, which are attached from only one side of the cell membrane. The method of the present invention has been used to encapsulate true integral membrane proteins with high structural fidelity inside reverse micelles.

Applications of the general method of the present invention also extend to proteins considered to be metastable or those prone to aggregation. The confining space of the reverse micelle can be used to induce conformational specificity on such proteins and reduce aggregation (Peterson, R.W., Anbalagan, K., Tommos, C., and Wand, A.J. 2004. J. Am. Chem. Soc. 126: 9498-9499). Furthermore, proteins that function in cells by anchoring to cellular membranes via an acyl chain (membrane-anchoring proteins) have been shown to adopt a folded, native anchored structure as determined by NMR $^{15}$N-HSQC.

The proteins should already have been subjected to some level of purification prior to the application of the solubilization method of the present invention. Purification of proteins can be achieved using the methods commonly known in the art. For the purpose of NMR or other spectroscopic studies, proteins should be greater than about 90% pure, preferably greater than about 95% pure.

One advantage of first immobilizing the protein on a solid support is that the loading, or quantity, of protein need not be dependent on the volume of the water phase, in contrast to the method of direct addition to the reverse micelle solution, where prior concentration of aqueous solutions of proteins is required. The initial concentration of the protein in the aqueous phase can be very low since the resin does the concentrating work. The present method also avoids harsh methods of dehydration such as lyophilization, where protein unfolding may occur, especially for large multidomain proteins or integral membrane proteins. One key advantage of the present method is that the target concentration of the protein in the final reverse micelle solution can be established up front by binding the appropriate quantity of protein to the resin, and eluting or extracting the known quantity of protein into a known volume of the reverse micelle organic phase. High levels of protein extraction are achieved and concentrated protein reverse micelle solutions are obtained. In some embodiments the concentrations obtained are between about 0.1 mM and about 0.3mM. In other embodiments lower concentrations may also be obtained. The more concentrated protein reverse micelle solutions are particularly suited to NMR analysis.

Protein immobilization on an ion-chromatography resin makes use of an active group or ion exchange group, with a specific charge. The charge on the protein, which is a function of the buffer pH, is what promotes binding and thus immobilization of the protein on the resin. This is the standard technique used in protein purification. In the context of the present invention, the charge of the ion exchange group must be taken into account when selecting the surfactant used for extraction into the reverse micelle, as well as the buffer conditions used to immobilize the protein. The surfactant head group should possess a matching charge to the resin ion exchange group. As an example, the cationic resin DEAE-Sepharose has a positively charged ion exchange group and can be matched to hexadecyl trimethylammonium bromide, also known as cetyl trimethylammonium bromide (CTAB), which has a positively charged head group. The resin carboxymethyl sepharose (CM-Sepharose) has a negatively charged ion-exchange group, and can be matched with the surfactant sodium bis(2-ethylhexyl)sulfosuccinate (AOT) with a negative charge on its head group.

Solid supports useful in the present invention are generally ion exchange resins, in which protein adsorption is moderated by changes in the magnitude of coulombic interactions between the protein and resin. For example, a resin that is an anion exchange resin has positively charged ionexchange groups (possible ion exchange types are Q, DEAE, and ANX). Examples of the resin matrix material include agarose or cellulose, for example, Q-sepharose, Q-sephacel, and Q-agarose.

A resin that is a cation exchange resin has negatively charged ion-exchange groups (possible ion exchange types are S, SP, and CM). The resin matrix material includes agarose or cellulose, for example, S-sepharose, S-sephacel, and S-agarose.

A metal chelate affinity resin may also be used in the present invention. The metal ions used in the coordination chemistry can be $Zn^{2+}$, $Ni^{2+}$, $Co^{2+}$, $Ca^{2+}$, $Cu^{2+}$, or $Fe^{3+}$. Such resins are commonly used to adsorb histag proteins. Such resins include, for example, NTA-agarose, HisPur®, or Talon® resins. The functional group is considered to be cationic for these resins.

Reversed-phase resins can also be used in the methods of the present invention. Typical binding groups in this class of resin are, for example, alkyl chains with lengths of 2, 4, 8, and 18 carbons, or phenyl or cyanopropyl groups. The functional groups are not charged for these resins so any surfactant can be used. Binding of the protein to the resin is via hydrophobic interactions.

For protein manipulations, including immobilization on, and elution or extraction from an ion exchange resin, the aqueous phase is generally buffered in order to control the charge state of the resin, surfactant and protein, and to provide a stable environment for the protein.

For certain metastable or complex proteins, for example integral membrane proteins, as discussed above, immobilization may require a surfactant or carrier membrane detergent in the loading buffer which is not compatible with reverse micelle formation. Therefore a surfactant exchange step is required which substitutes a reverse micelle-forming surfactant for the loading surfactant. The surfactant exchange of the immobilized protein may be carried out in either aqueous or organic solvents. Appropriate organic solvents for surfactant exchange include, without limitation, methanol, ethanol, dichloromethane, and dimethylsulfoxide. Typical carrier membrane detergents that require exchange include without limitation n-Decyl-β-maltopyranoside (DM), n-Dodecylphosphocholine (DPC), and 1,2-Dimyristoyl-sn-glycero-3-phosphocholine (DMPC).

As described above, the hydration state of the protein must be reduced in order to control the quantity of water in the resulting reverse micelle solution. This reduction of hydration may be accomplished by physically draining away the water from the resin and evaporation of residual water at either atmospheric pressure or reduced pressure (vacuum). In addition, appropriate organic solvents, as described above, may be used to wash the immobilized protein on the resin to remove excess water without denaturing the protein. Suitable solvents include diethyl ether, carbon disulfide, and methylene chloride. These methods of dehydration are mild enough that the protein will maintain a hydration shell promoting maintenance of the native structure.

Reverse micelles will spontaneously form as transparent solutions in a low polarity liquid, and are thermodynamically stable assemblies of surfactant molecules organized around a water core. The size and stability of a reverse micelle is dependent upon the amount of water loading, defined as the molar ratio of water to surfactant, $W_o$. Water loadings have been described that yield stable reverse micelles of AOT in a variety of long and short chain alkane solvents large enough to accommodate proteins. Indeed, there have been reports that all of the three shortest alkane solvents will support formation of a single phase containing reverse micelles. Typically $W_o$ is in the range of about 5 to about 30, preferably about 10 to about 20, most preferably about 10.

Surfactants for both the reverse micelle formation and the surfactant exchange step include any surfactant capable of forming reverse micelles (inverted micelles) in a non-polar solvent. Such surfactants have a charged or uncharged polar head group and a hydrophobic tail. Charged surfactants can be either cationic, anionic, or zwitterionic depending on the protein and the ion exchange resin. Non-ionic surfactants, having an uncharged polar head group and a hydrophobic tail, may also be used in the present invention.

Cationic surfactants include, without limitation, hexadecyltrimethytammonium bromide (CTAB), dodecyltrimethylammonium bromide (DTAB), diheradecyldimethylammonium bromide (DHAB), didodecyldimethylammonium bromide (DDAB), sodium dodecyl sulfate (SDS), trioctylmethylammonium chloride (TOMAC), and dioctyldimethylammonium chloride (DODMAC). These surfactants can be used with resins that have positively charged or neutral binding groups.

Anionic surfactants include, without limitation, bis(2-ethylhexyl) sodium succinate (AOT), dioleyl phosphoric acid (DOLPA), di(2-ethylhexyl) phosphothionic acid (DEPTA), and di(2-ethylhexl)phosphoric acid (DEHPA). These surfactants can be used with resins that have negatively charged or neutral binding groups.

Non-ionic surfactants include, without limitation, the family consisting of polyethylene monoalkyl ethers ($C_iE_j$) such as tetraethylene glycol monododecyl ether ($C_{12}E_4$), triethylene glycol monododecyl ether ($C_{12}E_3$), and tetraethylene glycol monodecyl ether ($C_{10}E_4$). Other examples are Tween-20, Tween-40, Tween-80 and Triton X-100. These surfactants can be used with any resin since they are not charged.

Zwitterionic surfactants include, without limitation, *N,N*-dimethyldodecylamine *N*-oxide, (LDAO), *N*-dodecyl-*N,N*-dimethyl-3-ammonio-1-propanesulfonate (ZW3-12), and *N*-dodecyl-*N,N*-dimetyl-3-ammonio-1-butyrate. These surfactants can be used with any resin since both positive and negative charges are present.

Preferred surfactants for the purposes of the present invention include hexadecyl trimethylammonium bromide, dodecyl trimethylammonium bromide, sodium bis(2-ethylhexyl) sulfosuccinate, lauryldimethylamine oxide, and N,N-dimethyl-N,N-dihexadecyl ammonium bromide.

Surfactant concentrations will generally fall in the range of about 50 mM to about 300 mM, preferably in the range of about 100 mM to about 200 mM.

Reverse micelles comprise at least one surfactant as above surrounding an aqueous core in a non-polar solvent. Note that the method of the present invention also allows for alterations to the reverse micelle encapsulation conditions while in contact with the resin through the addition of other reagents until encapsulation occurs. Other reagents might include a co-surfactant such as hexanol, or other lipophilic alcohol. Many combinations of surfactants and non-polar solvents, with or without cosurfactants, are possible. A mixture of surfactants is also possible, with or without alcoholic co-surfactants. Three common surfactant mixes are

70% $C_{12}E_4$ /30% AOT

70% $C_{12}E_4$ / 25% AOT / 5% DTAB

50% CTAB / 50% DHAB plus co-surfactant hexanol

The latter requires a co-surfactant (hexanol); the others do not.

Co-surfactants are occasionally required for reverse micelle formation. Surfactants such as CTAB, DTAB, DHAB, DDAB, and LDAO all require the addition of a co-surfactant. The most common co-surfactant is hexanol; however, other lipophilic alcohols such as butanol, pentanol, heptanol, octanol, and decanol are also suitable. Cosurfactant concentrations will generally fall in the range of about 5% to about 15% by volume. Preferably the cosolvent is hexanol, in a preferred concentration range of about 8% to about 12% by volume.

Reverse micelles are formed in non-polar solvents. Short to medium chain alkanes are commonly used as the solvents. Examples of useful solvents include, without limitation, methane, ethane, propane, butane, pentane, hexane, heptane, octane, nonane, and decane. Branched alkanes such as iso-octane can also be used. In addition, non-polar supercritical solvents such as carbon dioxide, xenon, and argon are appropriate solvents. Another class of suitable solvents includes halogenated hydrocarbons, for example and without limitation, chlorofluorocarbons (CFCs) and hydrochlorofluorocarbons (HCFCs). The non-polar solvent can also consist of a mixture of any of the above.

A principle embodiment of the present invention is a general method for the direct reverse micelle solution extraction of a protein that has been immobilized on an ion exchange or other resin. While not wishing to be bound by any particular theory, the extraction likely involves the change in the dielectric constant of the bulk solvent on going from the aqueous phase to a liquid alkane or other non-polar phase. This phase change alters the energetics of the charge-charge interactions such that it becomes more favorable for the protein to partition into the reverse micelle rather than remain bound to the column while bathed in organic solution. The method of the present invention may provide the solution to a series of problems plaguing the protein sample preparation process, especially for integral membrane proteins, which have presented a unique challenge for the reverse micelle method.

Proteins solubilized in reverse micelle solution are readily analyzed by a variety of physical methods, preferably spectroscopic methods. Examples of useful spectroscopic methods include, without limitation, fluorescence, UV-Vis, IR and NMR spectroscopy. As described in the Background of the Invention section, NMR spectroscopy is particularly useful for determining the tertiary solution phase structure and dynamics of proteins dissolved in reverse micelle solutions of the present invention. NMR techniques are also useful for studying small molecule binding, ligand binding, and protein-protein interactions of the protein reverse micelle solutions of the present invention. US Patent No. 6,198,281 describes the NMR analysis of proteins in reverse micelle solution.

To observe ligand or small molecule binding in solution, the NMR spectrum of the ligand-free protein is compared with that of the ligand-bound protein. When the ligand or small molecule binds to a protein, the signals corresponding to the protein may move, broaden, or disappear. Measuring changes in chemical shifts and hydrogen exchange rates serves to elucidate the dynamics of ligand binding and determine critical residues for ligand-protein interactions. Naturally occurring protein ligands, other naturally occurring molecules, for example cholesterol, and other small molecules, including totally synthetic compounds, may be analyzed in this way for binding to the protein of interest.

The method of the present invention may also be used to screen for macromolecule complex formation with other proteins of interest, to characterize their exchange regime and protein-protein interaction patterns, and to study dynamic and structural properties of protein-protein complexes.

## EXAMPLES

Examples of specific embodiments of the present invention are provided for the purpose of further describing the invention, and without intending the invention to be limited thereto. One skilled in the art might conceive of additional embodiments which would be encompassed by the present invention.

## Example 1

This example is illustrative of the solubilization of a typical soluble protein in a reverse micelle solution according to the present invention.

The protein Flavodoxin C55A mutant from *Cyanobacterium anabaena,* was used as a model protein. This protein has a yellow color making it very easy observe the protein by visual inspection. The resin selected as the immobilization substrate was DEAE-Sepharose with the ion exchange group diethylaminoethyl that possesses a positive charge. The surfactant used in the extraction was CTAB with a positively charged trimethylammonium head group.

A bed volume of approximately 200 $\mu$l of DEAE-Sepharose resin was added to a 1.7 ml conical tube. The resin was washed with an aliquot of 500 $\mu$l of the binding buffer, 50 mM Tris, pH 8, the tube was centrifuged, and the wash solution drawn off. This was repeated three times. A fourth aliquot was added that contained 200 $\mu$M Flavodovin. The solution was mildly agitated to promote binding of Flavodoxin to the resin. The sample was again centrifuged and the supernatant removed. The supernatant did not contain significant quantities of Flavodoxin indicating that the protein was now immobilized on the resin. Excess water was displaced by eluting the resin with diethyl ether. The resin was allowed to dry at room temperature.

A reverse micelle stock solution was prepared using 200 mM CTAB, 10% (v/v) of the cosurfactant hexanol in the alkane pentane. The aqueous phase in the reverse micelle was unbuffered water ($W_o$ = 10). An al-

iquot of 600 µl of this stock was added to the tube and allowed to remain in contact with the resin for five minutes. At the end of this period the reverse micelle organic phase was drawn off the resin. By visual inspection it appeared that very little Flavodoxin remained bound to the resin indicating a very high degree of extraction into the reverse micelle.

**Example 2**

This example is a further example of the solubilization of a typical water soluble protein in a reverse micelle solution according to the present invention.

The protein Cytochrome c from horse heart was used as a model protein. This protein has a deep red color making it very easy to monitor the progression of the experiment by visual inspection. The resin for this experiment was CM-Sepharose, which has a negatively charged carboxymethyl ion exchange group. The surfactant chosen was AOT since it has a negatively charged head group.

The resin bed volume was approximately 200 µl. The resin was washed three times with 500 µl of binding buffer, 25 mM sodium phosphate, pH 7. A fourth aliquot of buffer containing 200 µM Cytochrome c was added to the resin and the tube was agitated to promote binding. The sample was centrifuged, and the supernatant drawn off. There was some slight amount of Cytochrome c left in the supernatant, but the overall binding to the resin was high.

A reverse micelle stock solution was prepared using 200 mM AOT in pentane. The aqueous buffer in the reverse micelle phase was 50 mM acetate, pH 5, $W_o$=10. An aliquot of 600 µl of the reverse micelle solution was added to the reaction tube and allowed to remain in contact for 15 minutes. The extraction appeared to take longer than for Flavodoxin, but extraction into the reverse micelle was also very high.

**Example 3**

This example describes the solubilization of a typical integral membrane protein in a reverse micelle solution according to the present invention.

The MacKinnon construct for the transmembrane domain (Doyle, D.A., Cabral, J.M., Pfuetzner, R.A., Kuo, A.L., Gulbis, J.M., Cohen, S.L., Chait, B.T., and MacKinnon, R. 1998. Science 280: 69-77) of the potassium channel KcsA, a 68 kDa homotetramer from the soil bacteria *Streptomyces Lividans* (Schrempf, H., Schmidt, O., Kummerlen, R., Hinnah, S., Muller, D., Betzler, M., Steinkamp, T., and Wagner, R. 1995. EMBO J. 14: 5170-5178) is used as the model integral membrane protein. This protein has a C-terminal His-tag fusion that is useful for the method described herein. Polyhistidine-tag resin is selected as the solid support. The surfactant used for the exchange step as well as for the reverse micelle formation is CTAB with the positively charged head group matched to the binding group of the resin.

A bed volume of approximately 400 µl of the polyhistidine-tag resin is placed in a polyethylene spin column. The resin is preloaded with the metal $Co^{2+}$. Further equilibration of the resin is performed by three additions of 1-ml aliquots of the binding buffer (50 mM Tris, 150 mM NaCl, pH 7.5) to the column and centrifugation at 3,000 rpm for one minute in a clinical centrifuge to remove the buffer. The KcsA protein is previously purified by established protocols; stock solution is approximately 25 uM monomer. The stabilizing membrane detergent consists of 40 mM n-Decyl-β-maltopyranoside (DM). The aqueous phase buffer is the same as the binding buffer.

The protein is bound by passing 8 ml of the stock solution by gravity through the resin, in order to achieve a target concentration of 200 µm of monomer bound to the column. The column is again centrifuged at 3,000 rpm for one minute to remove any remaining stock solution from the resin. The eluent from the column is checked by UV-Vis spectroscopy at 280nm to confirm that binding to the resin is essentially complete.

The CTAB stock solution used for the exchange step is 10 mM CTAB in the binding buffer. An aliquot of 10 ml of the exchange solution is passed through the column by gravity, followed by centrifugation at 3,000 rpm for one minute to remove excess buffer. At this point the original detergent DM membrane is fully exchanged to the CTAB membrane. Excess buffer is removed by adding two 1 ml aliquots of diethyl ether to the column followed by centrifugation at 3,000 rpm for one minute. A final centrifugation is performed to remove any residual fluid. The protein is now ready for extraction into the reverse micelle.

The stock reverse micelle solution consists of 150 mM CTAB, 10% hexanol (v/v) in pentane, with an aqueous buffer consisting of 50 mM potassium phosphate, 50 mM KCl, pH 6.0, $W_o$ =10. The spin column is capped to prevent the reverse micelle solution from flowing through the column. A 1 ml aliquot of the reverse micelle solution is added to the resin. Mild agitation of the resin is performed several times during the extraction period of 15 minutes to promote mixing. The column is centrifuged at 3,000 rpm for one minute to remove the reverse micelle solution.

Although the present invention has been described with respect to a specific preferred embodiment thereof, various changes and modifications may be suggested to one skilled in the art, and it is intended that the present invention encompass such changes and modifications as fall within the scope of the following claims.

**Claims**

1. A method for solubilizing a water soluble protein in a reverse micelle comprising:

   (a) providing said protein;

(b) immobilizing said protein on a solid support in an aqueous phase;

(c) reducing the hydration of said protein; and

(d) extracting said protein off said support into a reverse micelle organic phase by contacting said support plus said protein with said organic phase.

2. A method for solubilizing an integral membrane protein in a reverse micelle comprising:

(a) providing said protein;

(b) immobilizing said integral membrane protein and its carrier membrane detergent molecules on a solid support in an aqueous phase;

(c) exchanging said carrier membrane detergents with a surfactant capable of forming reverse micelles;

(d) washing away excess surfactants and reducing the hydration of said protein on said solid support; and

(e) extracting said protein off said support into a reverse micelle organic phase by contacting said support plus said protein with said organic phase.

3. The method according to claim 1 wherein said protein is a membrane-anchoring protein or a water-soluble protein that aggregates in solution.

4. The method according to claim 1 or 2 wherein the solid support is an ion-exchange resin and/or wherein the solid support is any medium capable of reversibly binding and releasing the protein from the aqueous phase.

5. The method according to claim 1 or 2 where the reverse micelle includes at least one surfactant component selected from the group consisting of hexadecyl trimethylammonium bromide, dodecyl trimethylammonium bromide, sodium bis(2-ethylhexyl) sulfosuccinate, lauryldimethylamine oxide, and N,N-dimethyl-N,N-dihexadecyl ammonium bromide or where the reverse micelle includes at least one surfactant component selected from the group consisting of hexadecyl trimethylammonium bromide, dodecyl trimethylammonium bromide, sodium bis(2-ethylhexyl) sulfosuccinate, lauryldimethylamine oxide, N,N-dimethyl-N,N-dihexadecyl ammonium bromide, and a co-surfactant consisting of an alcohol, preferably hexanol.

6. The method according to claim 1 or 2 wherein said organic phase can be any solvent capable of supporting reverse micelles and/or wherein said organic phase is selected from the group consisting of decane, nonane, octane, iso-octane, heptane, hexane, pentane, butane, propane, ethane, and isomers or mixtures thereof.

7. The method according to claim 2 where the surfactant exchange in step (c) is conducted using a surfactant selected from the group consisting of hexadecyl trimethylammonium bromide, dodecyl trimethylammonium bromide, sodium bis(2-ethylhexyl) sulfosuccinate, lauryldimethylamine oxide, and N,N-dimethyl-N,N-dihexadecyl ammonium bromide.

8. The method according to claim 1 or 2 wherein said solubilized protein is suitable to be analyzed by NMR spectroscopy or to other spectroscopic analysis such as fluorescence, UV-Vis, or IR spectroscopy, and/or wherein said solubilized protein is suitable to be used to study small molecule binding and/or ligand binding and/or protein-protein interactions by spectroscopic techniques.

9. The method according to claims 1 and 2 wherein the reverse micelle phase further comprises components not required for reverse micelle formation, but which interact with the protein.

10. The method according to claim 9 wherein said components are selected from the group consisting of small molecules, natural protein ligands, synthetic protein ligands, oligonucleotides, and other natural membrane components.

11. The method according to claim 1 or 2 wherein the protein further includes bound or associated small molecules, natural protein ligands, synthetic protein ligands, and/or other natural membrane components.

12. The method according to claim 1 or 2 whereby the reduction of hydration is carried out at reduced pressure and/or by addition of an organic solvent to displace water and/or by draining away water from said solid support.

13. The method according to claim 2 whereby the bulk solvent carrying the surfactant used for exchanging with detergents is water.

14. The method according to claim 2 whereby the bulk solvent carrying the surfactant used for exchanging with detergents is an organic solvent.

15. The method of claims 1 or 2 wherein the protein concentration in the reverse micelle solution is at least about 0.1 mM.

**Patentansprüche**

1. Verfahren zum Solubilisieren eines wasserlöslichen Proteins in einer reversen Mizelle, welches umfasst, dass

   (a) das Protein bereitgestellt wird;
   (b) das Protein auf einem festen Träger in einer wässrigen Phase immobilisiert wird;
   (c) die Hydratation des Proteins verringert wird; und
   (d) das Protein aus dem Träger in eine organische Phase einer reversen Mizelle durch Inkontaktbringen des Trägers sowie des Proteins mit der organischen Phase extrahiert wird.

2. Verfahren zur Solubilisierung eines integralen Membranproteins in einer reversen Mizelle, welches umfasst, dass

   (a) das Protein bereitgestellt wird;
   (b) das integrale Membranprotein und seine Trägermembrandetergensmoleküle auf einem festen Träger in einer wässrigen Phase immobilisiert werden;
   (c) die Trägermembrandetergentien mit einem zur Bildung reverser Mizellen fähigem Surfactant ausgetauscht werden;
   (d) überschüssige Surfactants weggewaschen werden und die Hydratation des Proteins auf dem festen Träger verringert wird; und
   (e) das Protein aus dem Träger in eine organische Phase einer reversen Mizelle durch Inkontaktbringen des Trägers sowie des Proteins mit der organischen Phase extrahiert wird.

3. Verfahren nach Anspruch 1, wobei das Protein ein Membranverankerungsprotein oder ein wasserlösliches Protein ist, das in Lösung aggregiert.

4. Verfahren nach Anspruch 1 oder 2, wobei der feste Träger ein Ionenaustauscherharz und / oder wobei der feste Träger ein beliebiges Medium ist, das fähig ist, das Protein reversibel zu binden und aus der wässrigen Phase freizusetzen.

5. Verfahren nach Anspruch 1 oder 2, wo die reverse Mizelle mindestens einen Surfactantbestandteil enthält, der aus der Gruppe ausgewählt wird, die aus Hexadecyltrimethylammoniumbromid, Dodecyl-trimethylammoniumbromid, Natrium-bis(2-ethylhexyl)sulfosuccinat, Lauryldimethylaminoxid, und N,N-dimethyl-N,N-Dihexadecylammoniumbromid besteht, oder wo die reverse Mizelle mindestens einen Surfactantbestandteil, der aus der Gruppe ausgewählt ist, die aus Hexadecyl-trimethylammoniumbromid, Dodecyltrimethylammoniumbromid, Natrium-bis(2-ethylhexyl)sulfosuccinat, Lauryldimethylaminoxid, N,N-dimethyl-N,N-Dihexadecylammoniumbromid besteht, und ein Co-Surfactant, bestehend aus einem Alkohol, vorzugsweise Hexanol, enthält.

6. Verfahren nach Anspruch 1 oder 2, wobei die organische Phase jegliches Lösungsmittel sein kann, das fähig ist, reverse Mizellen zu unterstützen, und / oder wobei die organische Phase aus der Gruppe bestehend aus Decan, Nonan, Octan, iso-Octan, Heptan, Hexan, Pentan, Butan, Propan, Ethan, und Isomeren oder Gemischen davon, ausgewählt wird.

7. Verfahren nach Anspruch 2, wobei der Surfactantaustausch in Schritt (c) unter Verwendung eines Surfactants durchgeführt wird, das aus der Gruppe ausgewählt wird, die aus Hexadecyltrimethylammoniumbromid, Dodecyltrimethylammoniumbromid, Natrium-bis(2-ethylhexyl)sulfosuccinat, Lauryldimethylaminoxid, und N,N-Dimethyl-N,N-Dihexadecylammoniumbromid besteht.

8. Verfahren nach Anspruch 1 oder 2, wobei das solubilisierte Protein geeignet ist, durch NMR-Spektroskopie analysiert zu werden oder für andere spektroskopische Analysen wie zum Beispiel Fluoreszenz, UV-Vis, oder IR-Spektroskopie, und / oder wobei das solubilisierte Protein geeignet ist, dazu verwendet zu werden, "small molecule"-Bindung und / oder Ligandenbindung und / oder Protein-Protein-Wechselwirkungen durch spektroskopische Techniken zu untersuchen.

9. Verfahren nach den Ansprüchen 1 und 2, wobei die Phase der reversen Mizelle darüberhinaus Bestandteile aufweist, die nicht für die Bildung von reversen Mizellen erforderlich sind, aber welche mit dem Protein wechselwirken.

10. Verfahren nach Anspruch 9, wobei die Bestandteile aus der Gruppe bestehend aus "small molecules", natürlichen Protein-Liganden, synthetischen Protein-Liganden, Oligonukleotiden, und anderen natürliche Membranbestandteilen, ausgewählt werden.

11. Verfahren nach Anspruch 1 oder 2, wobei das Protein darüberhinaus gebundene oder assoziierte "small molecules", natürliche Protein-Liganden, synthetische Protein-Liganden, und / oder andere natürliche Membranbestandteile enthält.

12. Verfahren nach Anspruch 1 oder 2, wobei die Verringerung der Hydratation unter vermindertem Druck und / oder durch Zugabe eines organischen Lösungsmittels um Wasser zu verdrängen und / oder durch Abzug von Wasser von dem festen Träger durchgeführt wird.

13. Verfahren nach Anspruch 2, wobei der Großteil des Lösungsmittels, das das Surfactant zum Austausch mit Detergentien führt, Wasser ist.

14. Verfahren nach Anspruch 2, wobei der Großteil des Lösungsmittels, das das Surfactant zum Austausch mit Detergentien führt, ein organisches Lösungsmittel ist.

15. Verfahren nach den Ansprüchen 1 oder 2, wobei die Proteinkonzentration in der reversen Mizellen-Lösung mindestens etwa 0,1 mM ist.

**Revendications**

1. Procédé de solubilisation d'une protéine hydrosoluble dans une micelle inverse, comprenant les étapes consistant à :

   (a) fournir ladite protéine ;
   (b) immobiliser ladite protéine sur un support solide dans une phase aqueuse ;
   (c) réduire l'hydratation de ladite protéine ; et
   (d) extraire ladite protéine dudit support dans une phase organique de micelle inverse par contact dudit support plus de ladite protéine avec ladite phase organique.

2. Procédé de solubilisation d'une protéine intégrale de membrane dans une micelle inverse, comprenant les étapes consistant à :

   (a) fournir ladite protéine ;
   (b) immobiliser ladite protéine intégrale de membrane et ses molécules de détergent de membrane support sur un support solide dans une phase aqueuse ;
   (c) échanger lesdits détergents de membrane support par un tensioactif capable de former des micelles inverses ; et
   (d) retirer par lavage l'excès de tensioactifs et réduire l'hydratation de ladite protéine sur ledit support solide ; et
   (e) extraire ladite protéine dudit support dans une phase organique de micelle inverse par contact dudit support plus de ladite protéine avec ladite phase organique.

3. Procédé selon la revendication 1, dans lequel ladite protéine est une protéine à ancrage membranaire ou une protéine hydrosoluble qui s'agrège en solution.

4. Procédé selon la revendication 1 ou 2, dans lequel le support solide est une résine échangeuse d'ions et/ou dans lequel le support solide est un milieu capable de se lier de façon réversible et de libérer la protéine de la phase aqueuse.

5. Procédé selon la revendication 1 ou 2, dans lequel la micelle inverse inclut au moins un composant tensioactif sélectionné dans le groupe composé de l'hexadécyl-bromure de triméthylammonium, du dodécyl-bromure de triméthylammonium, du bis (2-éthylhexyl)sulfosuccinate de sodium, de l'oxyde de lauryldiméthylamine et du N,N-diméthyl-N,N-dihexadécyl-bromure d'ammonium, ou dans lequel la micelle inverse inclut au moins un composant tensioactif sélectionné dans le groupe composé de l'hexadécyl-bromure de triméthylammonium, du dodécyl-bromure de triméthylammonium, du bis (2-éthylhexyl)sulfosuccinate de sodium, de l'oxyde de lauryldiméthylamine et du N,N-diméthyl-N,N-dihexadécyl-bromure d'ammonium et d'un co-tensioactif composé d'un alcool, de préférence de l'hexanol.

6. Procédé selon la revendication 1 ou 2, dans lequel ladite phase organique peut être tout solvant capable de supporter des micelles inverses et/ou dans lequel ladite phase organique est sélectionnée dans le groupe composé du décane, du nonate, de l'octane, de l'iso-octane, de l'heptane, de l'hexane, du pentane, du butane, du propane, de l'éthane, et des isomères ou mélanges de ceux-ci.

7. Procédé selon la revendication 2, dans lequel l'échange de tensioactif de l'étape (c) est réalisé en utilisant un tensioactif sélectionné dans le groupe composé de l'hexadécyl-bromure de triméthylammonium, du dodécyl-bromure de triméthylammonium, du bis(2-éthylhexyl)sulfosuccinate de sodium, de l'oxyde de lauryldiméthylamine, et du N,N-diméthyl-N,N-dihexadécyl-bromure d'ammonium.

8. Procédé selon la revendication 1 ou 2, dans lequel ladite protéine solubilisée est appropriée pour être analysée par spectroscopie RMN ou une autre analyse spectroscopique telle que la fluorescence, UV-Vis, ou la spectroscopie IR, et/ou dans lequel ladite protéine solubilisée est appropriée pour étudier la liaison d'une petite molécule et/ou la liaison d'un ligand et/ou des interactions protéine-protéine par des techniques spectroscopiques.

9. Procédé selon les revendications 1 et 2, dans lequel la phase de micelle inverse comprend en outre des composants qui ne sont pas nécessaires pour la formation de la micelle inverse mais qui interagissent avec les protéines.

10. Procédé selon la revendication 9, dans lequel lesdits composants sont sélectionnés dans le groupe composé de petites molécules, de ligands de protéine naturels, de ligands de protéine synthétiques, d'oligonucléotides et d'autres composants de membrane naturels.

**11.** Procédé selon la revendication 1 ou 2, dans lequel la protéine contient en outre de petites molécules liées ou associées, des ligands de protéine naturels, des ligands de protéine synthétiques et/ou d'autres composants de membrane naturels.

**12.** Procédé selon la revendication 1 ou 2, dans lequel la réduction de l'hydratation est réalisée à une pression réduite et/ou par addition d'un solvant organique pour déplacer l'eau et/ou par drainage de l'eau hors dudit support solide.

**13.** Procédé selon la revendication 2, dans lequel le solvant principal portant le tensioactif utilisé pour l'échange de détergents est de l'eau.

**14.** Procédé selon la revendication 2, dans lequel le solvant principal portant le tensioactif utilisé pour l'échange de détergents est un solvant organique.

**15.** Procédé selon la revendication 1 ou 2, dans lequel la concentration en protéine dans la solution de micelle inverse est au moins égale à environ 0,1 mM.

Figure 1

Panel A

Panel B

Alkane Solvent

Water

Protein

AOT     bis(2-ethylhexyl) sodium succinate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6198281 B

**Non-patent literature cited in the description**

- **CLORE, G.M. ; GRONENBORN, A.M.** *Nat. Struct. Biol.,* 1997, vol. 4, 849-853
- **WAGNER, G.** *Nat. Struct. Biol.,* 1997, vol. 4, 841-844
- **SORENSEN, O.W. ; EICH, G.W. ; LEVITT, M.H. ; BODENHAUSEN, G. ; ERNST, R.R.** *Prog. NMR Spectr.,* 1987, vol. 16, 163-192
- **KAY, L.E. ; CLORE, G.M. ; BAX, A. ; GRONENBOM, A.M.** *Science,* 1990, vol. 249, 411-414
- **MCINTOSH, L.P. ; DAHLQUIST, F.W.** *O. Rev. Biophys.,* 1990, vol. 23, 1-38
- **LEMASTER, D.M.** *Prog. NMR Spectr.,* 1994, vol. 26, 371-419
- **PERVUSHIN, K. ; RIEK, R. ; WIDER, G. ; WUTHRICH, K.** *Proc. Nat. Acad. Sci. USA,* 1997, vol. 94, 12366-12371
- **RIEK, R. ; PERVUSHIN, K. ; WUTHRICH, K.** *Trends Biochem. Sci.,* 2000, vol. 25, 462-468
- **TUGARINOV, V. ; MUHANDIRAM, R. ; AYED, A. ; KAY, L.E.** *J. Am. Chem. Soc.,* 2002, vol. 124, 10025-10035
- **WAND, A.J. ; EHRHARDT, M.R. ; FLYNN, P.F.** *Proc. Nat. Acad. Sci. USA,* 1998, vol. 95, 15299-15302
- **BABU, C.R. ; FLYNN, P.F. ; WAND, A.J.** *J. Am. Chem. Soc.,* 2001, vol. 123, 2691-2692
- **LEFEBVRE, B.G. ; LIU, W. ; PETERSON, R.W. ; LEFEBVRE, B.G. ; WAND, A.J.** *J. Magn. Reson.,* 2005, vol. 175, 158-162
- **PETERSON, R.W. ; LEFEBVRE, B.G. ; WAND, A.J.** *J. Am. Chem. Soc.,* 2005, vol. 127, 10176-10177
- **LEFEBVRE, B.G. ; LIU, W. ; PETERSON, R.W. ; VALENTINE, K.G. ; WAND, A.J.** *J. Magn. Reson.,* 2005, vol. 175, 158-162
- **PETERSON, R.W. ; POMETUN, M.S. ; SHI, Z. ; WAND, A.J.** *Protein Sci.,* 2005, vol. 14, 2919-2921
- **SHI, Z. ; PETERSON, R.W. ; WAND, A.J.** *Langmuir,* 2005, vol. 21, 10632-10637
- **CHEREZOV, V. ; ROSENBAUM DANIEL, M. ; HANSON MICHAEL, A. ; RASMUSSEN SOREN, G.F. ; THIAN FOON, S. ; KOBILKA TONG, S. ; CHOI, H.-J. ; KUHN, P. ; WEIS WILLIAM, I. ; KOBILKA BRIAN, K. et al.** *Science (New York,* 2007, vol. 318, 1258-1265
- **GOKLEN, K.E. ; HATTON, T.A.** *Biotechnol Progr,* 1985, vol. 1, 69-74
- **GOKLEN, K.E. ; HATTON, T.A.** *Abstr Pap Am Chem,* 1985, vol. S 190, 128
- **JOHNSTON, K.P. ; RANDOLPH, T. ; BRIGHT, F. ; HOWDLE, S.** *Science,* 1996, vol. 272, 1726
- **FRANK, S.G. ; ZOGRAFI, G.** *J. Coll. Interf. Sci.,* 1969, vol. 29, 27-35
- **GALE, R.W. ; FULTON, J.L. ; SMITH, R.D.** *J. Am. Chem. Soc.,* 1987, vol. 109, 920-921
- **FULTON, J.L. ; SMITH, R.D.** *Acs Sym Ser,* 1988, vol. 373, 91-107
- **FULTON, J.L. ; BLITZ, J.P. ; TINGEY, J.M. ; SMITH, R.D.** *J Phys Chem-Us,* 1989, vol. 93, 4198-4204
- **FULTON, J.L. ; BLITZ, J.P. ; TINGEY, J.M. ; SMITH, R.D.** *J Phys Chem-Us,* vol. 93, 4198-4204
- **SMITH, R.D. ; FULTON, J.L. ; BLITZ, J.P. ; TINGEY, J.M.** *J Phys Chem-Us,* 1990, vol. 94, 781-787
- **MATZKE, S.F. et al.** *Biotechnology and Bioengineering,* 1992, vol. 40, 91-102
- **MELO, E.P. et al.** *Chemistry and Physics of Lipids,* 2003, vol. 124, 37-47
- **PETERSON, R.W. ; ANBALAGAN, K. ; TOMMOS, C. ; WAND, A.J.** *J. Am. Chem. Soc.,* 2004, vol. 126, 9498-9499
- **DOYLE, D.A. ; CABRAL, J.M. ; PFUETZNER, R.A. ; KUO, A.L. ; GULBIS, J.M. ; COHEN, S.L. ; CHAIT, B.T. ; MACKINNON, R.** *Science,* 1998, vol. 280, 69-77
- **SCHREMPF, H. ; SCHMIDT, O. ; KUMMERLEN, R. ; HINNAH, S. ; MULLER, D. ; BETZLER, M. ; STEINKAMP, T. ; WAGNER, R.** *EMBO J.,* 1995, vol. 14, 5170-5178